(19) Europäisches Patentamt
European Patent Office
Office européen des brevets

(11) Publication number: 0 211 601
A2

# (12) EUROPEAN PATENT APPLICATION

(21) Application number: 86305824.4

(22) Date of filing: 29.07.86

(51) Int. Cl.⁴: A 61 K 37/36
A 61 K 47/00

(30) Priority: 30.07.85 US 760630

(43) Date of publication of application:
25.02.87 Bulletin 87/9

(84) Designated Contracting States:
AT BE CH DE FR GB IT LI LU NL SE

(71) Applicant: International Minerals And Chemical Corporation
P.O. Box 207 1401 South Third Street
Terre Haute Indiana 47808(US)

(72) Inventor: Blum, Aleksander
39 Lakeview Drive
Terre Haute Indiana 47803(US)

(72) Inventor: Sivaramakrishnan, Kallidaikurichi N.
4330 South 6th Street, Apt. 4
Terre Haute Indiana 47802(US)

(72) Inventor: Viswanathan, Ravi
6409 Santona Street, Apt. 1B1
Coral Gables Florida 33146(US)

(74) Representative: Holmes, Michael John et al,
Frank B. Dehn & Co. Imperial House 15-19 Kingsway
London WC2B 6UZ(GB)

## (54) Stabilization of growth promoting hormones.

(57) The present invention provides a growth promoting formulation for administration to an animal comprising a mixture of water, a growth promoting hormone and a block copolymer containing polyoxyethylene-polyoxypropylene units and having an average molecular weight of about 1,100 to about 40,000.

EP 0 211 601 A2

Croydon Printing Company Ltd

0211601

# STABILIZATION OF GROWTH PROMOTING HORMONES

This invention relates to a method for maintaining the fluidity of a growth promoting hormone and maintaining its biological activity upon administration to an animal. One major problem in the long term administration of growth promoting hormones is the loss of bioactivity due to the formation of insolubles in aqueous environments. The formation of these insolubles blocks tubing, membranes and various pumps of the implanted delivery devices. System failure almost always eventually results due to the formation of these insolubles.

Another problem associated with the administration of growth hormones is the frequency required and the resulting rise in labor costs necessitated thereby. Moreover, many animals utilized for human food consumption, particularly ruminants, such as cattle and sheep, are range-fed for extended periods of time prior to feedlot development, thus rendering administration by injection of the hormone virtually impossible and economically impractical.

Prior efforts to provide for the long-term release of active agents for medication to animals include incorporating the medication in a polymeric matrix whereby the active ingredient is leached into the surrounding tissues of the animal. One such formulation for sustaining the release of a biological agent is disclosed in U.S. Patent No. 3,737,521. This patent discloses a neck implant for releasing a physiologically active estrus-blocking progestational steroid hormone agent in the neck tissue of a fertile heifer comprising a solid essentially linear polyether urethane polymeric matrix containing the steroid hormone agent.

Another known sustained-release delivery means is disclosed in U.S. Patent No. 4,235,988. This patent describes a delivery means comprising a functionally effective amount of biologically active agent and a hydrophilic linear block polyoxyalkylene-polyurethane copolymer.

U.S. Patent Nos. 4,188,373 and 4,100,271 relate to polymeric pharmaceutical vehicles for delivery of pharmaceutically active chemical materials to mucous membranes. The pharmaceutical carriers are aqueous solutions of certain polyoxyethylene-polyoxypropylene condensates. These polymeric pharmaceutical vehicles are described as providing for increased drug absorbtion by the mucous membrane and prolonged drug action by a factor of two or more. Examples of such drugs which can be incorporated in the polyoxyethylene-polyoxypropylene vehicle include those pharmaceuticals directed to the treatment of ocular conditions. While the pharmaceuticals for the treatment of the eye are extensive, these pharmaceuticals are generally of a low molecular weight and do not suffer from the problems of loss of bioactivity and formation of insolubles upon administration to an animal. Other patents of interest include U.S. Patent Nos. 4,474,751, 4,474,752, 4,474,753 and 4,478,822. These patents are directed to various drug delivery systems comprising polymers which are tetra-substituted derivatives of ethylene diamine, propylene diamine, butylenediamine, pentylenediamine and hexylene diamine. The substituents are block copolymers of polyoxypropylene and polyoxyethylene. Examples of drugs which can be administered in this polymer delivery system include antibacterial substances, antiinflammatories, anti-parasitics, antiviral effective compounds and peptide drugs such as insulin and somatostatin. The majority of the above drugs have low

molecular weights and therefore are not subject to the formation of insolubles. These patents do not disclose a method for stabilization of growth promoting hormones. Therefore, there exists a need for a method of maintaining the fluidity of a growth promoting hormone and maintaining its biological activity for prolonged release upon administration.

## SUMMARY OF THE INVENTION

The present invention provides for a method for maintaining the fluidity of a growth promoting hormone and maintaining its biological activity upon administration to an animal which method comprises:

(a) forming a mixture comprising water, a growth promoting hormone and a block copolymer containing polyoxyethylene-polyoxypropylene units and having an average molecular weight from about 1,100 to about 40,000 wherein the block copolymer is in an amount sufficient to prevent the precipitation of the growth promoting hormone upon administration to the animal; and

(b) administering the admixture to the animal.

Also disclosed is a method for accelerating growth in animals which comprises administering to the animals a growth promoting formulation comprising an aqueous mixture containing an effective amount of a growth promoting hormone and a block copolymer containing polyoxyethylene-polyoxypropylene units. Surprisingly, these aqueous mixtures preserve the bioactivity and stability of the growth hormone and provide for a prolonged release rate upon administration to the animal.

DETAILED DESCRIPTION OF THE PREFERRED EMBODIMENT

The present invention provides a method of stabilizing growth promoting hormones which are administered to animals. Surprisingly, aqueous solutions of certain polyoxyethylene-polyoxypropylene block copolymers are useful as stabilizers for growth promoting hormone. Another advantage is the achievement of prolonged release rates of the growth hormone upon administration to the animal. The block copolymers for use in accordance with the present invention can be described by the following structural formulas:

Formula 1

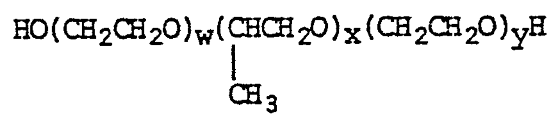

and

Formula 2

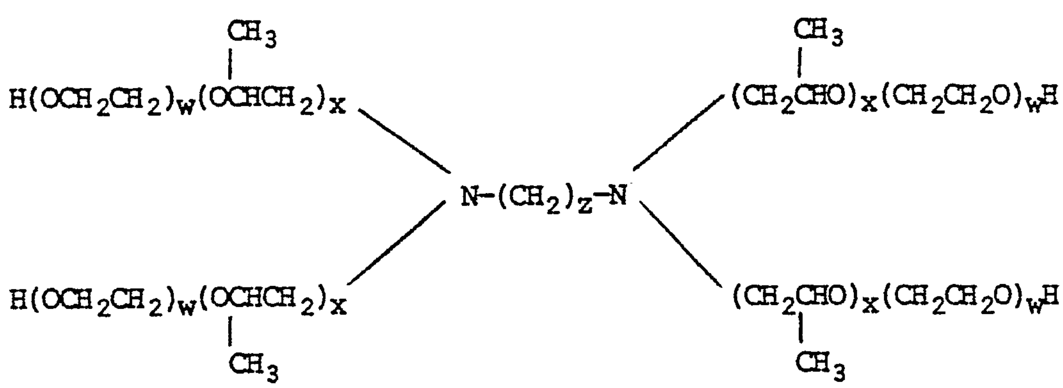

where z is an integer of from 2 to 6 and wherein the block copolymers of Formula 1 have an average molecular weight of from about 1,100 to about 15,500 and the block copolymers of Formula 2 have an average molecular weight of from about 1,600 to about 40,000 and w, x, and y are any integers within the above constraints.

The block copolymers of Formula 1 generally have an average molecular weight of from about 1,100 to about 15,500, with an average molecular weight of from 11,000 to about 13,000 being preferred. Generally, the polyoxyethylene units are at least 50% of the total number of units in the block copolymer of Formula 1. A particularly preferred block copolymer is when the polyoxyethylene unit is about 70% of the total number of units in the block copolymer. Block copolymers according to Formula 1 are commercially sold by BASF-Wyandotte Corporation of Wyandotte, Michigan under the trademark "PLURONIC". The "PLURONICS" are block copolymers generically classified as polyoxypropylene-polyoxyethylene condensates terminating in primary hydroxyl groups. They are formed by the condensation of propylene oxide into a propylene glycol nucleus followed by the condensation of ethylene oxide onto both ends of the polyoxypropylene base. Examples of PLURONIC" block copolymers include: Pluronic F-68, Pluronic F-77, Pluronic P-75, Pluronic P-65, Pluronic L-64, Pluronic F-87, Pluronic F-88, Pluronic F-98, Pluronic F-108 and Pluronic F-127. One particularly preferred Pluronic is Pluronic F-127. Other commercially available block copolymers classified under Formula 1 are those products sold under the trademark "GENAPOL" by Hoechst AG of Frankfurt, Germany.

The block copolymers of Formula 2 generally have an average molecular weight of from 1,600 to about 40,000, with an average molecular weight of from 15,000 to about 20,000 being preferred. Similar to the block copolymers of Formula 1, the polyoxyethylene units are at least 50% of the total number of units in the block copolymer of Formula 2. Preferably, the polyoxyethylene units are about 70% of the total number of units in the block copolymer. Block copolymers of Formula 2 are also

commercially available from BASF-Wyandotte Corporation. These block copolymers are sold under the trademark "Tetronic®".

The above polyoxyethylene-polyoxypropylene block copolymers must be present in an amount sufficient to stabilize the growth promoting hormone. Since other ingredients are also present in the formulation, such as water and any other optional compounds, the concentration of the block copolymer should also be high enough to result in a suitable gel matrix upon administration to the animal. The block copolymer is generally present in the growth hormone formulation in amounts from about 0.05 to about 50 percent by weight of the total aqueous growth promoting formulation, and preferably from about 1 to about 30 percent by weight of the total aqueous composition.

Aside from serving as a stabilizer for the growth hormone, these block copolymers provide excellent vehicles for the delivery of the growth hormone. One advantage of these block copolymers is that they are physiologically acceptable. Another advantage is their sol-gel transition temperatures. At temperatures at or below room temperature (30°C), aqueous solutions of block copolymers are in a liquid state. Above the temperatures, the aqueous solutions of block copolymers form a gel. Therefore, at lower temperatures a growth promoting formulation can be produced which is suitable for administration to the animal. However, after administration, the block copolymers form an excellent gel matrix for the prolonged release of the polypeptide.

Suitable growth promoting hormones include human, bovine, avian, ovine, equine and porcine growth promoting ormones. Preferably, the hormone is a porcine growth promoting hormone. The growth promoting hormone should be present in the aqueous mixture in an amount effective

to promote growth upon administration to the animal. While the amount of the growth promoting hormone may vary depending on the particular hormone, the type of animal and the desired results, the growth promoting hormone is generally present in amounts of from about .05 to about 10 percent by weight of the total aqueous mixture. Preferably, the growth promoting hormone is present in amounts of from about .10 to about 5 percent of the overall total weight of the aqueous mixture.

The growth hormones for use in the present invention can be derived by extraction and subsequent concentration techniques from the pituitary glands of various animals. Growth hormones which are produced by recombinant DNA methods are also suitable. The amino acid sequences of various hormones which are suitable in the present invention are known. For example, the amino acid sequence of human growth promoting hormones is described in an article by C.H. Li in Kirk-Othmer "Encyclopedia of Chemical Technology", 3rd E., Vol. 12, pp. 549-552. The amino acid sequence of bovine growth hormone is described in an article by R.P. Woychik, Nucleic Acid Res., 10, 7197 (1982). The amino acid sequence of ovine growth hormone is described in an article by C.H. Liu et al., Arch. Biochem. Biophys., 156, 493-508 (1973). The amino acid sequence of porcine growth hormone is described in an article by P.H. Seeburg et al., DNA, 2, 37, 45 (1983). All of the above references describing the amino acid sequences are hereby incorporated by reference in their entirety. In addition to the above, one can also use growth hormones that have been modified by excission of up to 12 amino acid residues from the amino ends of the amino acid sequences.

The block copolymer and the growth promoting hormone are dispersed in an aqueous solution. The

concentration of the water can vary widely depending on a number of factors such as the desired viscosity of the formulation prior to and after administration, the concentration of the growth hormone, temperature of preparation, etc. Generally, the water will be present in amounts of from about 50 to about 99.9 percent by weight of the total weight of the aqueous growth hormone formulation with from about 75 to about 95 percent by weight being preferred.

The aqueous growth promoting hormone formulation may optionally contain various adjuvants which further contribute to the prolonged release rate. Examples of such adjuvants include beeswax, aluminum monostearate and the like. Preferably, the adjuvant is aluminum monostearate.

While the present method involves an aqueous dispersion, one may optionally add various oils to assist in prolonging the release rate of the growth promoting hormone. Examples of such oils include mineral oils and vegetable oils such as sesame oil, peanut oil, soybean oil and the like.

The preparation of the aqueous formulations which are used in the present invention may be formed by simple mechanical mixing. The polyoxyethylene-polyoxypropylene copolymers are known to dissolve at reduced temperatures. Therefore, the preferred method of solubilization of the polymer includes dispersing the block copolymer in an aqeuous solution at temperatures below the sol-gel temperature of the solution of the copolymer. The aqueous mixture can then be stirred, sonicated or shaken to bring about a more rapid solubilization of the polymer in solution. After the aqueous solution of the polymer has been formed, the growth promoting hormone is added. While the above procedure is described as being preferred, the order of addition can be altered and

should be in no way deemed as limiting to the scope of the present invention. After the growth promoting hormone and the block copolymer have been dispersed in an aqueous solution, optional additives, such as buffers, salts, adjuvants, etc., may be added and dissolved.

Since the aqueous growth promoting hormone formulation is intended to be administered to an animal, the pH must be such that it is physiologically acceptable to the animal and does not contribute to the destabilization of the growth promoting hormone. Generally, the pH of an aqueous growth promoting formulation can range from about 5 to about 11 with the preferred pH range being from about 7 to about 10. The pH of the aqueous formulation can be adjusted by adding effective amounts of a pharmaceutically acceptable buffer to obtain the required pH. Suitable pharmaceutically acceptable buffers are generally known to those skilled in the art.

This invention additionally provides a method for effecting growth promotion in animals which comprises administering to the animal an effective amount of a prolonged release formulation comprising from about 0.05 to about 50 percent by weight of a polyoxyethylene-polyoxypropylene block copolymer having an average molecular weight of from about 1,100 to about 50,000 with from about .05 to about 10 percent by weight of a growth promoting growth hormone and from about 50 to about 99.9 percent of water. A preferred method according to the present invention comprises administering an effective amount of a prolonged release formulation comprising from about 1 to about 30 percent by weight of a polyoxyethylene-polyoxypropylene block copolymer having an average molecular weight of about from 11,000 to about 13,000 and about .5 to about 5 percent by weight of a porcine growth promoting hormone and from about 75 to

about 95 percent by weight of a phosphate buffered saline solution.

The present invention also provides for a growth promoting formulation having improved stability against the formation of insolubles comprising from about .05 to about 50 percent by weight of a block copolymer containing polyoxyethylene-polypropylene units and having an average molecular weight of from about 1,100 to about 40,000 with about .05 to about 10 percent by weight of a growth promoting hormone and from about 50 to about 99.9 percent by weight of water.

The growth promoting hormone formulations are for administration to an animal and preferably a ruminant. These formulation can be administered in a variety of ways. In one embodiment of the present invention, the aqueous solution is administered by subcutaneous or intramuscular injection. Another embodiment of the present invention is to administer the formulation to a reservoir of an implanted device having a means capable of delivering an effective amount of the aqueous polypeptide formulation. A number of delivery means which are implanted in the animals are known to those skilled in the art and are contemplated for use with the formulations of the present invention.

The present invention is illustrated in further detail by way of the following examples which, however, are not to be construed as limiting the scope thereof.

EXAMPLE 1

Isotonic physiological phosphate buffer (PBS) having a pH of 7.3 was prepared from sodium hydrogen phosphates (0.1 M phosphates) plus 0.2 percent $NaN_3$. Using this buffer, a solution of 18 percent by weight of PLURONIC F 127 was prepared by mechanical mixing. Then a

ca. 40 mg portion of porcine growth hormone (IMC Lot No. CF005-CDE-94.1) in a 50 ml sterile centrifuge tube was wetted with 100 µl of the solution. As a control, another ca. 40 mg portion of the same growth hormone was wetted with 100 µl of PBS solution containing no additives. The tubes were stored at 37°C. Following the 14 days at 37°C, each 50 ml tube was typically handled as follows. First, 40 ml of PBS was added to the tube (defined as start of solubilization time). Using a cup sonicator, the wetted material in the tube was effectively suspended/dissolved. The resulting suspension was subsequently vortexed during a period of standing at room temperature. The respective tube was centrifuged for ca. 5 min. X 1900 G (defined as the end of solubilization time). The resulting supernatant was nearly all removed by pipet and the undissolved residue suspended in 20 ml added water. Following centrifugation and supernatant removal, the residue in the tube was dried at room temperature in a Savant Speed Vac Concentrator to a final chamber pressure of ca. 150 millitorr. The pelleted dry residue was removed from the tube and its weight determined by the different weight of the tube.

It is apparent from the Table below that a significant decrease in the rate of formation of insolubles is achieved by use of the stabilizer of the present invention.

TABLE 1

| | PBS Solution | 18% by weight PLURONIC F127/ PBS Solution |
|---|---|---|
| rpGH | IMC Lot No. CF005-CDE94.1 | IMC Lot No. CF005-CDE94.1 |
| Days @ 37°C | 14 | 14 |
| Solubilization Time (hrs) | 1.5 | 1.5 |
| Insolubles as Wt % of Starting rpGH | 67 | 43 |

EXAMPLE 2

In an effort to demonstrate the ability of the stabilizers of the present invention to preserve high levels of bioactivity of growth hormones, the following experiments were conducted.

A first solution (Solution A) was prepared which consisted of a phosphate buffered saline solution (PBS) containing recombinant porcine growth hormone (IMC Lot No. 01-00A.94.1) in a concentration of 1 mg/ml of PBS. A second solution (Solution B) was prepared which was identical to the first solution but additionally contained 5 weight percent of PLURONIC F 127. Each solution was then placed in a peristaltic pump and continuously circulated at room temperature for a period of 24 hours. The two solutions were then removed from the circulation device.

Each solution was then diluted in order to administer varying concentrations of the growth hormone solutions to rats (See Table 2 below for concentrations). As another control, an uncirculated solution (Solution C) identical to solution A was prepared and then diluted.

Each diluted solution was administered to a number of rats. Only one injection was given. The weight gain of each rat was then determined after 10 days following the administration of the solution. Table 2 below lists the dosage of the growth hormone that was administered, the percentage of weight gain and the relative potency of the growth hormone.

The data in the table below demonstrates that the relative potency of a circulated growth hormone which contains a stabilizer (Solution B) is higher than circulated unstabilized solutions (Solution A) and is equal to uncirculated growth hormone solutions (Solution C).

TABLE 2

| SOLUTION | DOSE (µg) | NO. OF RATS | % WEIGHT GAIN | | % RELATIVE POTENCY* |
|---|---|---|---|---|---|
| | | | MEAN | STD. DEV. (+/-) | |
| Solution A (rpGH+PBS) | 6 | 10 | 7.1 | 3.2 | 21 |
| | 12 | 10 | 10.2 | 3.4 | |
| | 24 | 10 | 12.0 | 3.3 | |
| Solution B (rpGH+5% PLUORINIC/PBS) | 6 | 5 | 9.2 | 2.1 | |
| | 12 | 9 | 9.9 | 5.3 | 33 |
| | 24 | 10 | 15.5 | 1.9 | |
| Solution C (rpGH+PBS) | 6 | 9 | 10.2 | 3.3 | |
| | 12 | 10 | 11.4 | 4.1 | 33 |
| | 24 | 10 | 13.4 | 3.6 | |

* Value derived from all dosage ranges of Solutions, i.e., A, B, and C.

Claims:

1. A growth promoting formulation for administration to an animal comprising a mixture of water, a growth promoting hormone and a block copolymer containing polyoxyethylene-polyoxypropylene units and having an average molecular weight of about 1,100 to about 40,000.

2. A formulation as claimed in claim 1 wherein said block copolymer is selected from copolymers of formula (1)

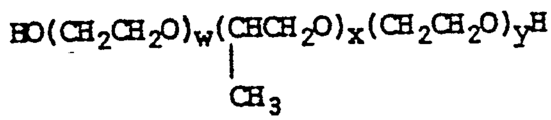

(1)

and formula (2)

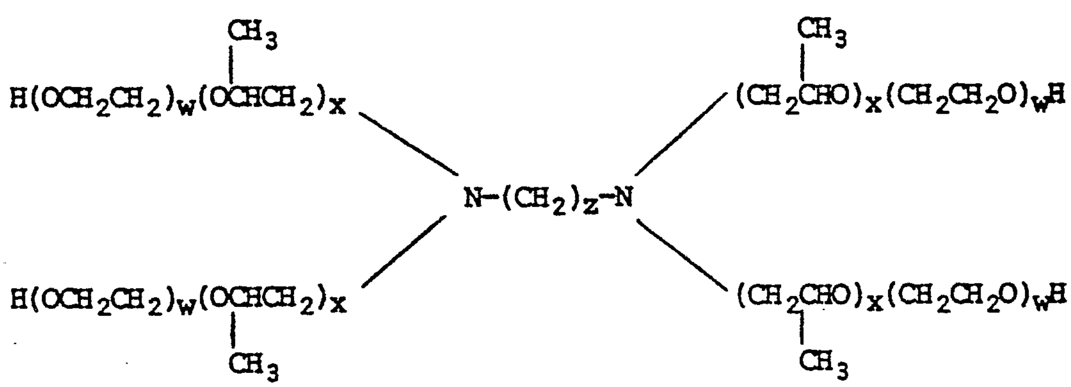

(2)

where z is 2 to 6 and wherein the block copolymers of formula (1) have an average molecular weight of about 1,100 to about 15,500 and the block copolymers of formula (2) have an average molecular weight of about 1,600 to about 40,000 and w, x, and y are any integers within the above constraints.

3. A formulation as claimed in claim 1 or claim 2 wherein said block copolymer is present in an amount of about 0.05 to about 50 percent by weight based on the total weight of the formulation.

4. A formulation as claimed in any one of claims 1 to 3 wherein the number of polyoxyethylene units in said block copolymer is at least 50 percent of the total units.

5. A formulation as claimed in any one of claims 1 to 4 wherein said block copolymer has an average molecular weight of about 11,000 to about 12,000.

6. A formulation as claimed in any one of claims 1 to 5 wherein said growth promoting hormone is present in an amount of about 0.05 to about 10 percent by weight based on the total weight of the formulation.

7. A formulation as claimed in any one of claims 1 to 6 wherein water is present in an amount of about 50 to about 99.9 percent by weight based on the total weight of the formulation.

8. A formulation as claimed in any one of claims 1 to 7 wherein said growth promoting hormone is a human, bovine, avian, porcine, equine or ovine growth promoting hormone.

9. A formulation as claimed in claim 8 wherein said growth promoting hormone is a porcine growth promoting hormone.

10. A formulation as claimed in any one of claims 1 to 9 which additionally comprises one or more components selected from oils, adjuvants and buffers.

11. A formulation as claimed in any one of claims 1 to 10 having a pH of about 5 to about 11.

12. A method for promoting growth of an animal which comprises administering to the animal an effective amount of a formulation as claimed in any one of claims 1 to 11.

13. A method as claimed in claim 12 wherein said formulation is administered via a reservoir of a drug delivery device which is implanted in the animal.

14. A method as claimed in claim 12 wherein said formulation is administered by subcutaneous or intramuscular injection.